# EUROPEAN PATENT SPECIFICATION

(11) **EP 3 099 294 B1**
(45) Date of publication and mention of the grant of the patent: **01.08.2018**
(21) Application number: 15742755.0
(22) Date of filing: 29.01.2015
(51) Int. Cl.: A61K 9/48, A61K 31/34

(54) **PHARMACEUTICAL COMPOSITION INCLUDING 5-{4-(AMINOSULFONYL)PHENYL}-2,2-DIMETHYL-4-(3-FLUOROPHENYL)-3(2H)-FURANONE AND CAPSULE FORMULATION INCLUDING THE PHARMACEUTICAL COMPOSITION**
PHARMAZEUTISCHE ZUSAMMENSETZUNG MIT 5-{4-(AMINOSULFONYL)}PHENYL}-2,2-DIMETHYL-4-(3-FLUORPHENYL)-3(2H)-FURANON UND KAPSELFORMULIERUNG MIT DER PHARMAZEUTISCHEN ZUSAMMENSETZUNG
COMPOSITION PHARMACEUTIQUE COMPRENANT DE LA 5-{4-(AMINOSULFONYL)PHÉNYL}-2,2-DIMÉTHYL-4-(3(FLUOROPHÉNYL)-3(2H)-FURANONE ET CAPSULE COMPRENANT LA COMPOSITION PHARMACEUTIQUE

(30) Priority: 29.01.2014 KR 20140011315
(43) Date of publication of application: 07.12.2016
(73) Proprietor: Crystalgenomics Inc., Seongnam-si, Gyeonggi-do 463-400 (KR)
(72) Inventor: KIM, Byung-Ha, Seoul 143-751 (KR); AHN, Sik Il, Gwangju-si Gyeonggi-do 464-895 (KR); PARK, Jae-Yeon, Seoul 137-887 (KR); KIM, Tae Ryong, Seoul 151-812 (KR); CHO, Joong Myung, Seoul 138-791 (KR); RO, Seonggu, Seoul 138-740 (KR)
(74) Representative: Schiweck, Weinzierl & Koch Patentanwälte Partnerschaft mbB
(86) International application number: PCT/KR2015/001002
(87) International publication number: WO 2015/115853

(56) References cited:
- WO-A1-00/61571
- WO-A2-2007/052937
- KR-A- 20100 096 512
- US-A- 5 536 752
- US-A1- 2004 242 640
- US-A1- 2005 222 251
- DATABASE WPI Section Ch, Week 201401 Thomson Scientific, London, GB; Class A96, AN 2013-M57979 XP002772438, IM H T; KIM K S; KIM Y I; PARK J H; PARK S J; WOO J S: "Pharmaceutical composition useful for preventing and/or treating e.g. inflammatory disease and solid cancer, comprises particle comprising 4-3-(3-fluorophenyl)-5,5-dimethyl-4-oxa-4, 5-dihydrofuran-2-ylbenzenesulfonamide", -& KR 2013 0078147 A ((HANM) HANMI PHARM CO LTD) 10 July 2013 (2013-07-10)

## Description

### Technical Field

The present invention relates to a pharmaceutical composition including 5-{4-(aminosulfonyl)phenyl}-2,2-dimethyl-4-(3-fluorophenyl)-3(2H)-furanone and a capsule formulation including the pharmaceutical composition. More specifically, the present invention relates to a pharmaceutical composition including 5-{4-(aminosulfonyl)phenyl}-2,2-dimethyl-4-(3-fluorophenyl)-3(2H)-furanone that is useful as a non-steroidal anti-inflammatory drug due to its good stability, high dissolution rate, improved content uniformity, and excellent pharmacokinetic properties, and a capsule formulation including the pharmaceutical composition.

### Background Art

Prostaglandins are known to play an important role in causing inflammation. Prostaglandins are produced from arachidonic acid by cyclooxygenase (hereinafter abbreviated as "COX"). The activity of COX is suppressed to inhibit the synthesis of prostaglandins, particularly, PGE2, PGG2, and PGH2, resulting in the treatment of inflammation.

Two COX isoenzymes, COX-1 and COX-2, are known. COX-1 is inherently found in the gastrointestinal tract and kidney and is assumed to maintain physiological homeostatic functions, including gastrointestinal integrity and renal functions. Inhibition of COX-1 activity may cause life-threatening toxicities, such as ulcers and hemorrhage in the gastrointestinal tract. In contrast, COX-2 is induced by inflammatory stimuli and is known to be responsible for the development of inflammation.

COX-2 inhibitors are assumed to possess a broad spectrum of therapeutic activities as well as anti-inflammatory, analgesic, and antipyretic activities. For example, inhibition of COX-2 is known to prevent the onset of cancers, particularly colorectal cancer [J. Clin. Invest., 99, 2254 (1997)], can apply to the treatment of chronic neurodegenerative diseases, such as Alzheimer's disease [Neurology, 48, 626 (1997)], and is also known to be useful in the reduction of infarct volume accompanied by a stroke [J. Neuroscience, 17, 2746 (1997)].

Conventional non-steroidal anti-inflammatory drugs (NSAIDs), such as indomethacin, naproxen, ketoprofen, ibuprofen, piroxicam, and diclofenac, inhibit both COX-1 and COX-2 to show gastrointestinal toxicities together with anti-inflammatory efficacy. Furthermore, such NSAIDs have fatal toxicities, such as hemorrhage and ulcers, arising from the inhibition of COX-1, limiting their clinical use. Thus, selective COX-2 inhibitors are useful as therapeutic agents against inflammation and diseases accompanied by inflammation without causing gastrointestinal toxicities, which are common during long-term use of conventional NSAIDs.

4,5-Diaryl-3(2H)-furanone derivatives have recently been reported as selective inhibitors against COX-2 (Korean Patent No. 10-0495389). KR 2013 0078147 A discloses pharmaceutical compositions for preventing and/or treating inflammatory diseases comprising 5-{4-(aminosulfonyl)phenyl}-2,2-dimethyl-4-(3-fluorophenyl)-3(2H)-furanone. The D90 of the drug particles is of 50 micrometers or less, preferably of 25 micrometers or less. Example 1 of KR 2013 0078147 A refers to a capsule comprising the above drug with a D90 of 20.5 micrometers, the diluent microcrystalline cellulose and the lubricant talc. When the furanone derivatives are used to prepare pharmaceutical compositions, they are required to have high dissolution rate, good flowability, optimum mass variation, and improved content uniformity. The present inventors have found that a specific furanone derivative meets the requirements. Based on this finding, the present inventors have succeeded in preparing a pharmaceutical composition including the furanone derivative and a capsule formulation including the pharmaceutical composition and finally arrived at the present invention.

### Disclosure of Invention

### Technical Problem

It is one object of the present invention to provide a pharmaceutical composition comprising 5-{4-(aminosulfonyl)phenyl}-2,2-dimethyl-4-(3-fluorophenyl)-3(2H)-furanone, with high dissolution rate, good flowability, optimum mass variation, and improved content uniformity.

It is another object of the present invention to provide a capsule formulation including the pharmaceutical composition.

### Solution to Problem

According to one aspect of the present invention, there is provided a pharmaceutical composition according to claim 1 including (i) the compound of Formula 1: 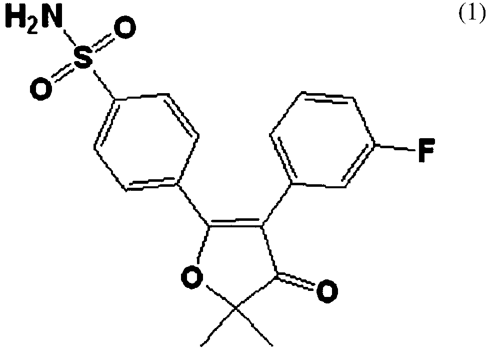 or a pharmaceutically acceptable salt thereof having a 50% volume particle diameter (d_{(0.5)}) of 3 µm to 9 µm, (ii) a pharmaceutically acceptable diluent, and (iii) a pharmaceutically acceptable lubricant.

According to another aspect of the present invention, there is provided a capsule formulation according to claim 8 including the pharmaceutical composition.

### Advantageous Effects of Invention

The pharmaceutical composition including 5-{4-(aminosulfonyl)phenyl}-2,2-dimethyl-4-(3-fluorophenyl)-3(2H)-furanone according to the present invention has the advantages of good stability, high dissolution rate, improved content uniformity, and excellent pharmacokinetic properties. Due to these advantages, the pharmaceutical composition of the present invention is effective in treating inflammation or pain.

### Brief Description of Drawings

Fig. 1 is a graph showing the results of differential scanning calorimetry (DSC) for a crystalline form prepared in Preparative Example 1.
Fig. 2 is a graph showing the results of differential scanning calorimetry (DSC) for a crystalline form prepared in Preparative Example 2.
Fig. 3 is a graph showing the results of differential scanning calorimetry (DSC) for a crystalline form prepared in Preparative Example 3.
Fig. 4a and 4b graphically show the dissolution rates of crystalline forms prepared in Preparative Examples 1 and 2 at different revolution numbers of 50 rpm (Fig. 4a) and 100 rpm (Fig. 4b).
Fig. 5 is a graph showing the dissolution rates of mixtures of crystalline forms prepared in Preparative Examples 1 to 7.
Fig. 6a and 6b show the results of X-ray diffraction analysis for crystalline forms prepared in Preparative Examples 1 (Fig. 6a) and 2 (Fig. 6b) after storage under different conditions.
Fig. 7 is a graph showing the pharmacokinetic properties of crystalline forms prepared in Preparative Examples 1 and 2 in rats.
Fig. 8a and 8b graphically show the dissolution rates of crystalline forms of Example 1 and Comparative Examples 1 and 2 with different eluting solutions.
Figs. 9a to 9c are chromatograms of formulations including a crystalline form of Example 1, as analyzed by HPLC after storage under light stress conditions; peaks marked with ★ indicate that related substances created under the light stress conditions exceeded the respective reference standards defined by related substance test methods.
Fig. 10 is a graph showing the particle size distributions of formulations produced in Examples 2 to 6.

### Mode for the Invention

The present invention will now be described in detail.

The present invention provides a pharmaceutical composition according to claim 1 including (i) the compound of Formula 1: 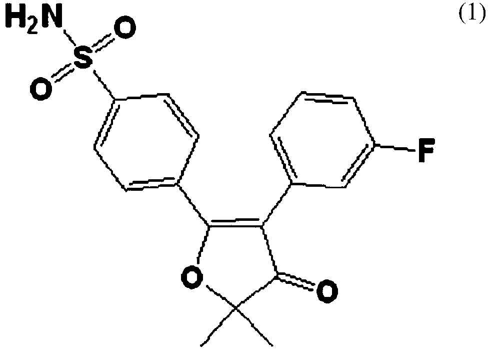 or a pharmaceutically acceptable salt thereof having a 50% volume particle diameter (d_{(0.5)}) of 3 µm to 9 µm, (ii) a pharmaceutically acceptable diluent, and (iii) a pharmaceutically acceptable lubricant.

The compound of Formula 1 is used as an active ingredient in the pharmaceutical composition of the present invention. The compound of Formula 1 is a selective COX-2 inhibitor whose chemical name is "5-{4-(aminosulfonyl)phenyl}-2,2-dimethyl-4-(3-fluorophenyl)-3(2H)-furanone". The compound of Formula 1 is known to have reduced gastrointestinal toxicities and be effective against inflammatory diseases, inflammation-associated diseases, pain, solid cancers, angiogenesis-associated diseases, Alzheimer's disease, attacks, convulsions, strokes, and epilepsy over conventional NSAIDs (see Korean Patent No. 10-0495389).

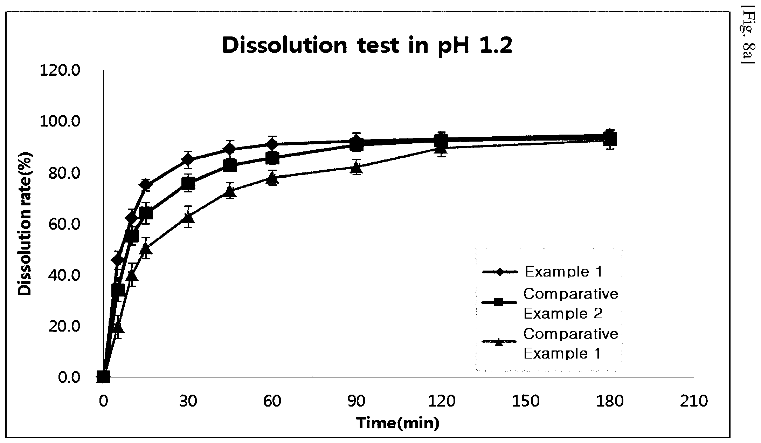

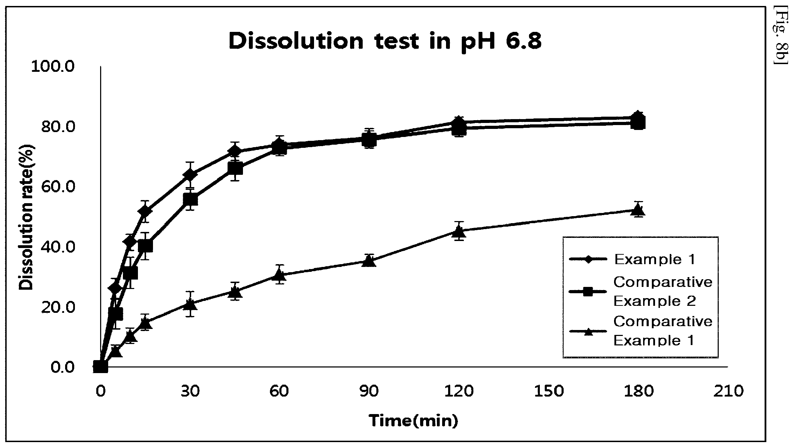

The compound of Formula 1 is characterized by having a 50% volume particle diameter (d_{(0.5)}) of 3 µm to 9 µm and, optionally, a 90% volume particle diameter (d_{(0.9)}) of 10 µm to 100 µm. The 50% volume particle diameter (d_{(0.5)}) means the particle diameter at which, when the volume of the particles is integrated in order from the smaller particle diameter side, the cumulative frequency of the volume distribution reaches 50% of the total volume. The 90% volume particle diameter (d_{(0.9)}) means the particle diameter at which, when the volume of the particles is integrated in order from the smaller particle diameter side, the cumulative frequency of the volume distribution reaches 90% of the total volume. The compound of Formula 1 is used in an amount of 0.5 to 20% by weight, based on the total weight of the pharmaceutical composition. Due to the use of a smaller amount, the 50% volume particle diameter (d_{(0.5)}) of 3 µm to 9 µm, and, optionally, the 90% volume particle diameter (d_{(0.9)}) of 10 µm to 100 µm, content uniformity of the compound of Formula 1 is easier to ensure when trituration is implemented using a diluent and a further improvement in the dissolution rate of the compound of Formula 1 is attained (Figs. 8a and 8b). The (d_{(0.5)}) may be 3 µm to 8 µm in some embodiments, 4 µm to 9 µm in further embodiments, and 4 µm to 8 µm in additional embodiments. The (d_{(0.9)}) may be 10 µm to 80 µm in some embodiments. 10 µm to 50 µm in further embodiments, and 10 µm to 20 µm in additional embodiments.

The compound of Formula 1 exists in crystalline form A, crystalline form G or a mixture thereof.

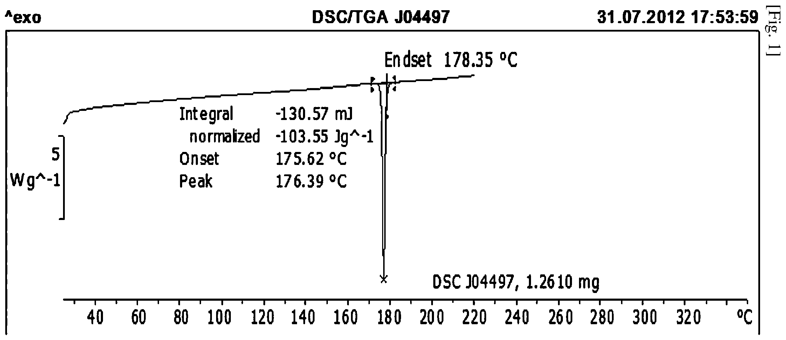

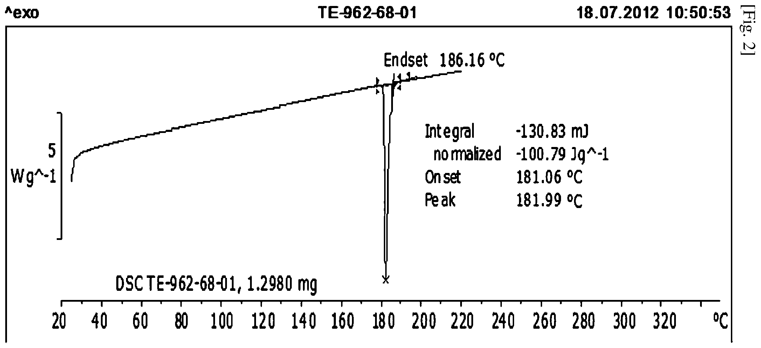

According to the results of experiments conducted by the present inventors, the crystalline form A has the results of X-ray diffraction analysis shown in Table 1 and the differential scanning calorimetry (DSC) profile shown in Fig. 1. The crystalline form G has the results of X-ray diffraction analysis shown in Table 2 and the differential scanning calorimetry (DSC) profile shown in Fig. 2.

The present inventors obtained crystalline forms B to F by recrystallization of the crystalline form A from suitable solvents, such as t-butyl methyl ether, isopropyl alcohol, methyl alcohol, ethyl alcohol, and acetonitrile. However, the crystalline forms B to F tended to return to the crystalline form A during storage at 40 °C and 75% RH for 4 days. In contrast, the crystalline forms A and G were highly stable. Particularly, when the particles in the crystalline form A are present in a larger amount, specifically, the crystalline form A is present in an amount of 50% by weight, based on the total weight of the crystalline forms, a higher dissolution rate was obtained. Accordingly, it is preferred that the compound of Formula 1 includes at least 50% by weight of the crystalline form A, based on the total weight of the compound.

The states of the crystalline forms A and G are maintained stable during long-term storage under accelerated storage conditions.

The compound of Formula 1 may be used in an amount of 0.5 to 20% by weight, preferably 1% by weight, based on the total weight of the pharmaceutical composition.

The compound of Formula 1 may exist in the form of a pharmaceutically acceptable salt.

The pharmaceutical composition of the present invention includes a pharmaceutically acceptable diluent and a pharmaceutically acceptable lubricant in addition to the active ingredient.

The diluent may be used in an amount of 75 to 99% by weight, based on the total weight of the pharmaceutical composition. As the diluent, there may be mentioned, for example, silicified microcrystalline cellulose (e.g., silicified microcrystalline cellulose 50 or 90), microcrystalline cellulose, cellulose, lactose or a combination thereof (e.g., Cellactose® 80). The use of silicified microcrystalline cellulose is preferred.

The lubricant may be used in an amount of 0.1 to 5% by weight, preferably 1% by weight, based on the total weight of the pharmaceutical composition. As the lubricant, there may be mentioned, for example, talc or stearic acid. The use of talc is preferred.

The pharmaceutical composition of the present invention may further include one or more pharmaceutically acceptable additives commonly used in the pharmaceutical art, in addition to the diluent and the lubricant.

The pharmaceutical composition can be used for the prevention or treatment of inflammatory diseases, inflammation-associated diseases, pain, solid cancers, angiogenesis-associated diseases, Alzheimer's disease, attacks, convulsions, strokes or epilepsy. The pharmaceutical composition is preferably used for the prevention or treatment of inflammatory diseases, inflammation-associated diseases or pain.

The pharmaceutical composition of the present invention can be processed into various pharmaceutical formulations.

The formulations may be in the form of tablets, powders, granules, capsules, suspensions, inhalation sprays, and injectable solutions. The formulations are preferably capsules, more preferably hard capsules.

The pharmaceutical composition of the present invention may be administered via various routes, including but not limited to, orally, intravenously, subcutaneously, and by topical application.

The pharmaceutical composition of the present invention may be administered in a daily dose of 0.1 to 100 mg/kg body weight to a patient. The daily dose may vary depending on the indication, condition or state of the patient. The pharmaceutical composition of the present invention may be administered according to various schedules, such as once, twice, and three times a day, but is not limited to these schedules.

The present invention will be explained in detail with reference to the following examples, including test examples. However, these examples are provided for illustrative purposes only and are not intended to limit the scope of the invention.

### Preparative Example 1: Preparation of 5-{4-(aminosulfonyl)phenyl}-2,2-dimethyl-4-(3-fluorophenyl)-3(2H)-furanone and characterization of crystalline form thereof (crystalline form A)

5-{4-(Aminosulfonyl)phenyl}-2,2-dimethyl-4-(3-fluorophenyl)-3(2H)-furanone was prepared in accordance with the procedure described in Example 4 of Korean Patent No. 10-0495389.

Specifically, 4-bromo-2,2-dimethyl-5-4-(aminosulfonyl)phenyl-3(2H)-furanone (170 mg) was dissolved in 30 mL of toluene and 10 mL of ethanol. The solution was stirred. To the solution were added dropwise 25 mg of tetrakis(triphenylphosphine)palladium (0), 10 mL of a saturated aqueous solution of sodium bicarbonate, and 100 mg of 3-fluorobenzeneboronic acid. After stirring at 90 °C for 12 hr, the solvents were removed from the reaction solution under reduced pressure and the residue was extracted with water and dichloromethane. The organic layer was concentrated under reduced pressure and the residue was purified by column chromatography (hexane/ethyl acetate), yielding 120 mg of 5-{4-(aminosulfonyl)phenyl}-2,2-dimethyl-4-(3-fluorophenyl)-3(2H)-furanone as a solid.

### (1) X-ray diffraction (XRD) analysis

After the compound prepared in Preparative Example 1 was crystallized by a general crystallization method, its crystalline form was characterized by X-ray diffraction (XRD) analysis. The XRD analysis was performed using an Ultima III high-resolution X-ray diffractometer (Rigaku, Japan) with Cu radiation.

The experimental results are shown in Table 1.

### Table 1

**[Table 1]**

| The crystalline form of the compound prepared in Preparative Example 1 | |
|---|---|
| 2θ | Intensity (cps) |
| 8.40 | 7125 |
| 13.26 | 10050 |
| 14.02 | 2612 |
| 17.70 | 12200 |
| 18.48 | 10388 |
| 19.14 | 7400 |
| 19.84 | 5150 |
| 20.54 | 11750 |
| 22.72 | 2788 |
| 23.56 | 3100 |
| 27.62 | 3088 |

### (2) Differential scanning calorimetry (DSC)

The crystalline form of the compound prepared in Preparative Example 1 was analyzed by differential scanning calorimetry (DSC). The DSC analysis was performed using a DSC 823e (Mettler Toledo, Switzerland). About 1-2.3 mg of a sample of the crystalline form was placed on an aluminum pan and heated at a rate of 10 °C/min from 25 °C to 220 °C. The data were analyzed with the STARe v9.20 (Proteus®).

The experimental results are shown in Fig. 1.

The crystalline form of the compound prepared in Preparative Example 1 with the results of XRD and DSC analyses was called "crystalline form A".

### Preparative Example 2: Preparation of 5-{4-(aminosulfonyl)phenyl}-2,2-dimethyl-4-(3-fluorophenyl)-3(2H)-furanone and characterization of crystalline form thereof (crystalline form G)

The crystalline form of the compound prepared in Preparative Example 1 was changed using a DSC instrument (Q2000, TA Instruments, UK or DSC 823e, Mettler Toledo, Switzerland). Specifically, 5 mg of a sample of the crystalline form A was placed on an aluminum pan and subjected to a heating-isothermal-cooling cycle in a TA instrument to prepare a new crystalline form. The cycle consisted of five stages: heating at a rate of 10 °C/min from 25 °C to 180 °C (stage 1); maintenance at 180 °C for 5 min (stage 2); cooling at a rate of 10 °C/min from 180 °C to 25 °C (stage 3); maintenance at 25 °C for 1 min (stage 4); and heating at a rate of 10 °C/min from 25 °C to 170 °C (stage 5). Throughout the preparation of the crystalline form of the compound, nitrogen purging was maintained at 50 ml/min.

### (1) X-ray diffraction (XRD) analysis

The crystalline form of the compound prepared in Preparative Example 2 was characterized by X-ray diffraction (XRD) analysis. The XRD analysis was performed using an Ultima III high-resolution X-ray diffractometer (Rigaku, Japan) with Cu radiation.

The experimental results are shown in Table 2.

### Table 2

**[Table 2]**

| The crystalline form of the compound prepared in Preparative Example 2 | |
|---|---|
| 2θ | Intensity (cps) |
| 11.10 | 3112 |
| 12.66 | 8762 |
| 16.92 | 7812 |
| 18.26 | 18038 |
| 19.48 | 8288 |
| 20.80 | 9775 |
| 22.46 | 4775 |
| 24.02 | 5350 |
| 25.42 | 17138 |
| 27.76 | 4700 |

### (2) Differential scanning calorimetry (DSC)

The crystalline form of the compound prepared in Preparative Example 2 was analyzed by differential scanning calorimetry (DSC). The DSC analysis was performed using a DSC 823e (Mettler Toledo, Switzerland). About 1-2.3 mg of a sample of the crystalline form was placed on an aluminum pan and heated at a rate of 10 °C/min from 25 °C to 220 °C. The data were analyzed with the STARe v9.20 (Proteus®).

The experimental results are shown in Fig. 2.

The results of the XRD and DSC analyses confirm that the crystalline form of the compound prepared in Preparative Example 2 is quite different from the crystalline form A of the compound prepared in Preparative Example 1. The crystalline form of the compound prepared in Preparative Example 2 with the results of XRD and DSC analyses was called "crystalline form G".

### Preparative Example 3: Preparation and characterization of mixture of the crystalline forms (crystalline form A+crystalline form G) of 5-{4-(aminosulfonyl)phenyl}-2,2-dimethyl-4-(3-fluorophenyl)-3(2H)-furanone

The crystalline forms of Preparative Examples 1 and 2 were mixed in a weight ratio of 50:50 to prepare a mixture. The mixture was characterized to investigate whether the characteristics of the crystalline forms were maintained.

The mixture of the crystalline forms A and G was analyzed by differential scanning calorimetry (DSC). The DSC analysis was performed using DSC 200 F3 Maia® (NETZSCH). About 1-5 mg of a sample of the mixture was placed on an aluminum pan and heated at a rate of 20 °C/min from 25 to 100 °C and at a rate of 10 °C/min from 100 to 250 °C. The data were analyzed with the STARe v9.20 (Proteus®).

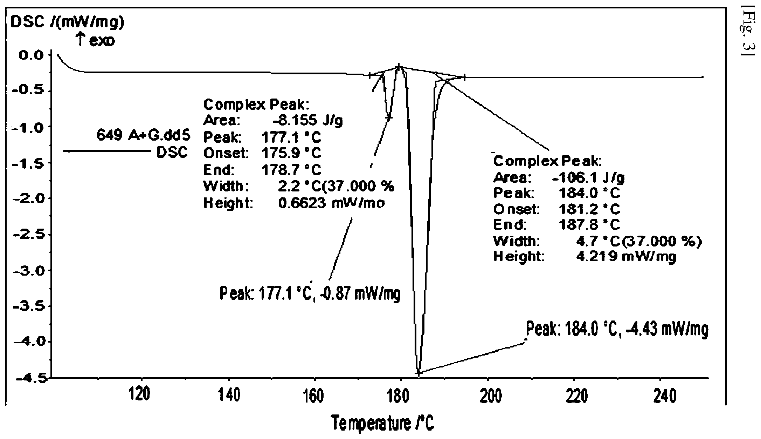

The experimental results are shown in Fig. 3.

As shown in Fig. 3, the DSC graph of the mixture of the crystalline forms prepared in Preparative Example 3 reveals the endothermic peaks corresponding to the crystalline forms of Preparative Examples 1 and 2. These results show that the crystalline forms A and G maintain their characteristics even when mixed.

### Test Example 1: Analysis of dissolution rates of the different crystalline forms

In this example, the dissolution rates of the crystalline forms of the compound of Formula 1 were examined. Specifically, each of the crystalline form A of Preparative Example 1 and the crystalline form G of Preparative Example 2 was filled in hard capsules and was then eluted in 900 ml of a pH 1.2 solution at different revolution numbers of 50 and 100 rpm and a temperature of 37±0.5 °C for 2 hr. The eluted particles were analyzed under the following HPLC conditions:

### <HPLC conditions>

Column: Hypurity C18, 250 X 4.6 mm, 5 µm or its equivalent column
Detector: UV absorption spectrometer (measured at 325 nm)
Injection volume: 100 µl
Flow rate: 1.5 ml/min
Column temperature: 30 °C
Mobile phase: A - acetonitrile, B - water, A:B = 60:40, v/v%
Analysis time: 5 min

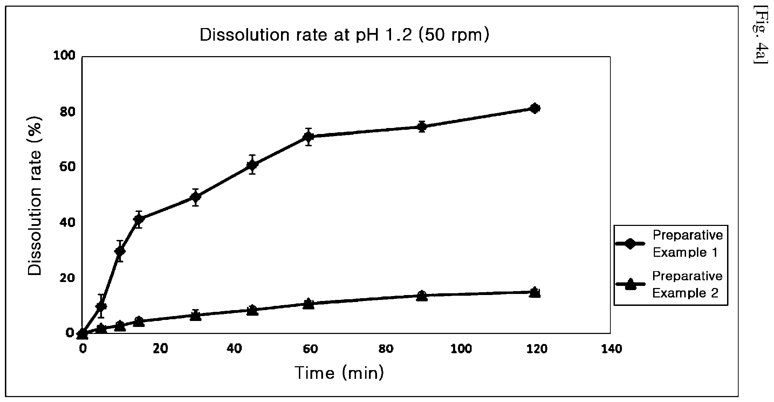

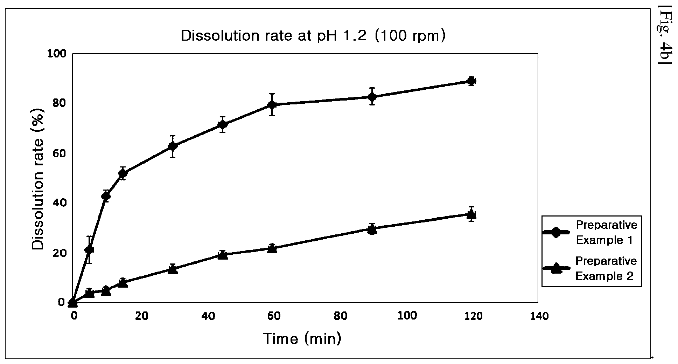

The experimental results obtained at revolution numbers of 50 rpm and 100 rpm are shown in Figs. 4a and 4b, respectively.

As can be seen from Figs. 4a and 4b, the crystalline form A showed higher dissolution rates than the crystalline form G at the two different revolution numbers. These results demonstrate that the crystalline forms of the compound of Formula 1 exhibit different dissolution rates and a large proportion of the crystalline form A would be advantageous in achieving a desired dissolution rate. Higher dissolution rates of formulations containing larger proportions of the crystalline form A were confirmed in Preparative Examples 4-7.

### Preparative Examples 4-7: Production of particles of mixtures containing the crystalline forms in different ratios

The crystalline form A of Preparative Example 1 and the crystalline form G of Preparative Example 2 were mixed in the ratios shown in Table 3. The dissolution rates of the mixtures were investigated.

### Table 3

**[Table 3]**

| | Preparative Example 4 | Preparative Example 5 | Preparative Example 6 | Preparative Example 7 |
|---|---|---|---|---|
| Crystalline form A (wt%) | 30 | 50 | 70 | 90 |
| Crystalline form G (wt%) | 70 | 50 | 30 | 10 |
| Total amount (%) | 100 | 100 | 100 | 100 |

### Test Example 2: Analysis of dissolution rates of the mixtures containing the crystalline forms in different ratios

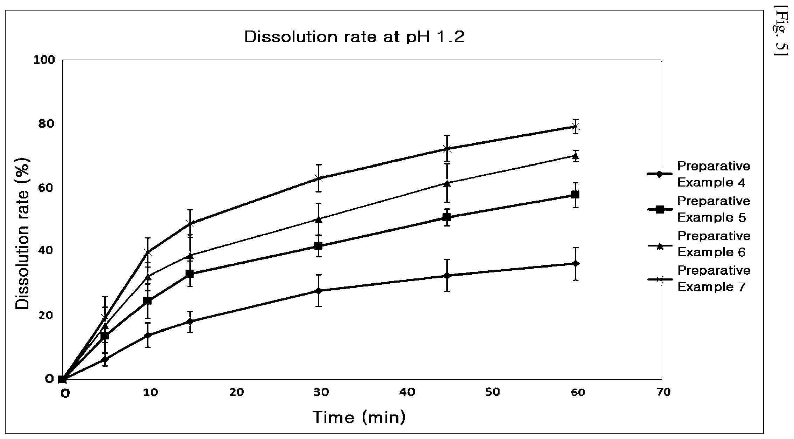

In this example, the dissolution rates of the particles of the mixtures of the crystalline forms A and G in different ratios were examined. Specifically, 2 mg of each of the mixtures prepared in Preparative Examples 4-7 was filled in a hard capsule and was then eluted in 900 ml of a pH 1.2 solution at a revolution number of 100 rpm and a temperature of 37±0.5 °C for 2 hr. The eluted particles were analyzed under the same HPLC conditions as described in Test Example 1. The experimental results are shown in Fig. 5.

As can be seen from Fig. 5, the dissolution rate increased with increasing proportion of the crystalline form A.

### Test Example 3: Analysis of stability of the crystalline forms

The crystalline forms of the compound of Formula 1 were evaluated for storage stability. The crystalline form A of Preparative Example 1 and the crystalline form G of Preparative Example 2 were filled in different hard capsules and stored under severe humidity conditions (25 °C/97% RH) and accelerated storage conditions (40 °C /75% RH) for 7 d. X-ray diffraction analysis was performed in accordance with the same method as described in Preparative Examples 1 and 2.

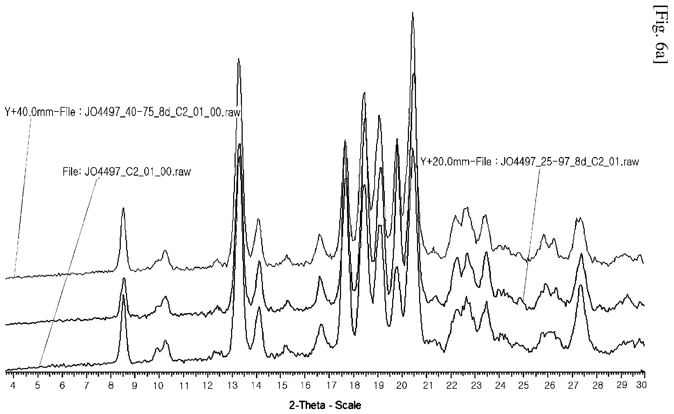

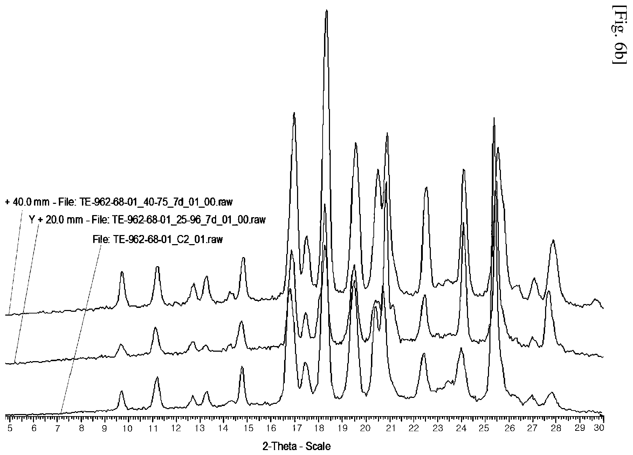

The results of analysis are shown in Figs. 6a and 6b.

As can be seen from Figs. 6a and 6b, the states of the crystalline forms A and G of the compound of Formula 1 were maintained stable under severe humidity conditions and accelerated storage conditions.

### Test Example 4: Analysis of pharmacokinetic properties of the crystalline forms

The pharmacokinetic properties of the different crystalline forms of the compound of Formula 1 were analyzed in vivo. About 5 mg of each of the crystalline form A of Preparative Example 1 and the crystalline form G of Preparative Example 2 was suspended in 10 mL of a 0.5% methylcellulose solution to produce a formulation for oral use. 6 week old male SD rats (Orient Bio. Inc., Korea) were divided into two groups. About 3 mL (10 mL/Kg) of the oral formulation was once administered orally to each rat and blood samples were drawn from the rat at predetermined intervals of 0.167, 0.5, 1.0, 2.0, 4.0, 6.0, 8.0, and 24.0 hr. The blood samples were used to analyze the pharmacokinetic parameters of the crystalline form.

The pharmacokinetic parameters of the crystalline forms were analyzed using Waters Quattro premier XE 2795 Alliance HT (Waters) under the following conditions: flow rate = 0.25 ml/min, column temperature = 40 °C, injection volume = 7 µL, and mobile phase = A: 1 mM ammonium acetate & 0.1% acetic acid (35%), B: ACN (65%). Linearity was established with 8 different standard concentrations.

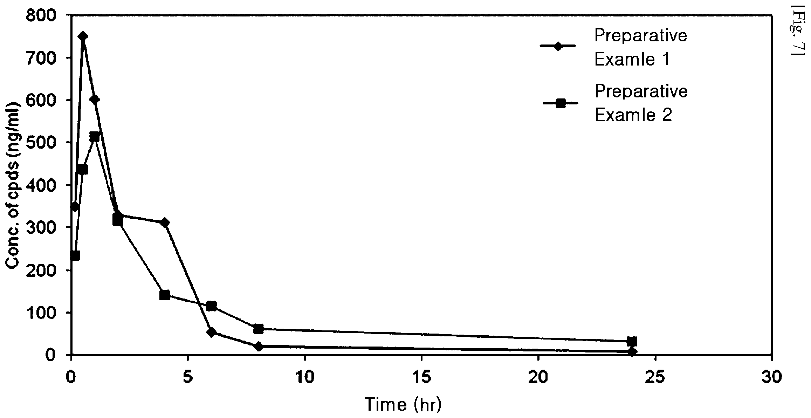

The oral formulations including the compound of Preparative Example 1 and the oral formulations including the compound of Preparative Example 2 were administered to the different rats. The blood levels of the compounds are graphically shown in Fig. 7. Cₘₐₓ (ng/mL), Tₘₐₓ (hr), and AUC (hr*ng/mL) were calculated from the graph and are shown in Table 4.

### Table 4

**[Table 4]**

| Parameter | Cₘₐₓ (ng/mL) | Tₘₐₓ (hr) | AUC (hr*ng/mL) |
|---|---|---|---|
| Preparative Example 1 | 750.582 | 0.5 | 2317.926 |
| Preparative Example 2 | 513.614 | 1.0 | 2416.835 |

As shown in Fig. 7, the crystalline form A of Preparative Example 1 showed higher in vivo dissolution rates than the crystalline form G of Preparative Example 2. As can be seen from the results in Table 4, the crystalline form A of Preparative Example 1 had higher Cₘₐₓ and Tₘₐₓ values than the crystalline form G of Preparative Example 2, demonstrating a faster efficacy of the crystalline form A of Preparative Example 1.

### Example 1 and Comparative Examples 1 and 2: Preparation of the crystalline form with different particle sizes

In order to compare the characteristics of the compound of Formula 1 as a drug depending on its particle size, the crystalline form was processed into different particle diameters by the following procedures.

### <Comparative Example 1>

The crystalline form prepared in Preparative Example 1 was called "Comparative Example 1".

### <Comparative Example 2>

The crystalline form of Comparative Example 1 was once pulverized using a mill (Jet mill, JE POWDER) under the following conditions: screw feeder = 7 rpm, agitator = 7 rpm, ejector pressure = 5.0 kg/cm², and line pressure = 3.5 kg/cm². The pulverized crystalline form was called "Comparative Example 2".

### <Example 1>

The crystalline form of Comparative Example 1 was once more pulverized using a mill (Jet mill, JE POWDER) under the following conditions: screw feeder = 7 rpm, agitator = 7 rpm, ejector pressure = 5.0 kg/cm², and line pressure = 3.5 kg/cm². The fine crystalline form was called "Example 1".

### Test Example 5: Analysis of the particle sizes and measurement of dissolution rates of the crystalline form with different particle sizes

### <5-1> Analysis of the particle sizes

The particle sizes of the crystalline form of Comparative Examples 1 and 2 and Example 1 were analyzed using a laser diffraction-based particle size analyzer (Mastersizer 2000®, Malvern). After each sample was fed into a dry module (Scirocco 2000®, Malvern) at a pressure of 2 bar, the 50% volume particle diameter (d_{(0.5)}) and 90% volume particle diameter (d_{(0.9)}) of the particles were measured. The experimental results are shown in Table 5.

### Table 5

**[Table 5]**

| | Particle size (µm) | | |
|---|---|---|---|
| | d_{(0.1)} | d_{(0.5)} | d_{(0.9)} |
| Comparative Example 1 | 40.55 | 136.49 | 527.04 |
| Comparative Example 2 | 5.15 | 32.17 | 83.54 |
| Example 1 | 1.75 | 5.98 | 15.23 |

As can be seen from the results in Table 5, the particles of Comparative Example 1 had a 50% volume particle diameter (d_{(0.5)}) of 136.49 µm and a 90% volume particle diameter (d_{(0.9)}) of 527.04 µm, the particles of Comparative Example 2 had a 50% volume particle diameter (d_{(0.5)}) of 32.17 µm and a 90% volume particle diameter (d_{(0.9)}) of 83.54 µm, and the particles of Example 1 had a 50% volume particle diameter (d_{(0.5)}) of 5.98 µm and a 90% volume particle diameter (d_{(0.9)}) of 15.23 µm. From these results, it could be confirmed that the crystalline forms of Comparative Examples 1 and 2 and Example 1 had different particle size distributions.

### <5-2> Analysis of dissolution rates of the crystalline form with different particle sizes

In this example, the dissolution rates of the crystalline form with different particle sizes were examined. The particles of Comparative Examples 1 and 2 and Example 1 were filled in different hard capsules (2 mg per capsule) and were then eluted in 900 ml of a pH 1.2 solution and 900 ml of a pH 6.8 solution at a revolution number of 100 rpm and a temperature of 37±0.5 °C for 3 hr. The eluted particles were analyzed under the same HPLC conditions as described in Test Example 1.

The results are shown in Figs. 8a and 8b.

As can be seen from Figs. 8a and 8b, the particles of Example 1 having a 50% volume particle diameter (d_{(0.5)}) of 3-9 µm and a 90% volume particle diameter (d_{(0.9)}) of 10-50 µm showed higher dissolution rates than the particles of Comparative Examples 1 and 2 whose 50% volume particle diameters and 90% volume particle diameters were outside the particle size distribution ranges of the particles of Example 1. These results show that a higher dissolution rate can be attained when the 50% volume particle diameter (d_{(0.5)}) and the 90% volume particle diameter (d_{(0.9)}) of the crystalline form A of the compound of Formula 1 are adjusted to the ranges of 3-9 µm and 10-50 µm, respectively.

### Test Example 6: Analysis of stability of the crystalline form of the compound of Formula 1

### <6-1> Temperature stability

The crystalline form A of Example 1 was filled in hard capsules (2 mg per capsule), packaged with PTP, and stored for 72 hr under the severe temperature conditions shown in Table 7. During the storage, the appearance of the crystalline form A, the retention time of the major peak, the amounts (%) of related substances, and the compound content were observed. The retention time of the major peak, the amounts of related substances, and the compound content were analyzed by HPLC under the following conditions. The results are shown in Table 6.

### <HPLC conditions for analysis of related substances>

Column: Hypurity C18, 250 X 4.6 mm, 5 m or its equivalent column
Detector: UV absorption spectrometer (measured at 241 nm)
Injection volume: 20 µl
Flow rate: 1.0 ml/min
Column temperature: 30 °C
Mobile phase: A - acetonitrile, B - 0.1% v/v trifluoroacetic acid (TFA) in water

| **Time (min)** | **Flow rate (mL/min)** | **A (%)** | **B (%)** |
|---|---|---|---|
| **0.00** | **1.0** | **38** | **62** |
| **30.00** | **1.0** | **38** | **62** |
| **35.00** | **1.0** | **90** | **10** |
| **45.00** | **1.0** | **90** | **10** |
| **45.01** | **1.0** | **38** | **62** |
| **50.00** | **1.0** | **38** | **62** |

### <HPLC conditions for analysis of the compound content >

Column: Hypurity C18, 250 X 4.6 mm, 5 m or its equivalent column
Detector: UV absorption spectrometer (measured at 325 nm)
Injection volume: 20 µl
Flow rate: 1.5 ml/min
Column temperature: 30 °C
Mobile phase: A - acetonitrile, B - water, A:B = 60:40, v/v%
Analysis time: 5 min
Diluent: water:acetonitrile = 50:50, v/v%

### Table 6

**[Table 6]**

| Packaging material | | PTP | | | |
|---|---|---|---|---|---|
| Stability test conditions(severe temperature conditions) | | 60±2°C60 ±5%RH24 hr | 80±2°C60 ±5%RH24 hr | 90±2°C60 ±5%RH24 hr | 90±2°C60 ±5%RH7 2 hr |

| Test item | Criteria | Results | | | |
|---|---|---|---|---|---|
| Appearance | White hard capsule containing white-pale yellow powder and marked with upper green CG649 | Unchange d | Unchange d | Unchange d | Unchange d |
| Peak retention time confirmation | Retention time (RT) of major peak (HPLC) | The same RT | The same RT | The same RT | The same RT |
| Amounts of related substances | Each < 0.3%, Total < 1.0% | 0.0%, 0.0% | 0.0%, 0.0% | 0.0%, 0.0% | 0.1%, 0.1% |
| Content | 95-105% | 103.0% | 101.5% | 101.8% | 101.1% |

As can be seen from the results in Table 6, the appearance of the crystalline form A remained unchanged, and no significant decrease in the content of the crystalline form A and no significant increase in the amount of related substances were observed under severe temperature conditions. These results demonstrate high stability of the crystalline form A under the temperature conditions.

### <6-2> Humidity stability

The crystalline form A of Example 1 was evaluated for humidity stability in the same manner as in Test Example <6-1>. The crystalline form A was filled in a hard capsule (2 mg per capsule) and stored under the severe humidity conditions shown in Table 7. Thereafter, the appearance of the crystalline form A, the retention time of the major peak, the amounts (%) of related substances, and the compound content were analyzed. The results are shown in Table 7.

### Table 7

**[Table 7]**

| Packaging material | | PTP | | |
|---|---|---|---|---|
| Stability test conditions(severe humidity conditions) | | 25±2°C90±5 %RH1 week | 25±2°C90±5 %RH2 weeks | 25±2°C90±5 %RH4 weeks |

| Test item | Criteria | Results | | |
|---|---|---|---|---|
| Appearance | White hard capsule containing white-pale yellow powder and marked with upper green CG649 | Unchanged | Unchanged | Unchanged |
| Peak retention time confirmation | Retention time (RT) of major peak (HPLC) | The same RT | The same RT | The same RT |
| Amounts of related substances | Each < 0.3%, Total < 1.0% | 0.1%, 0.1% | 0.1%, 0.1% | 0.1%, 0.1% |
| Content | 95-105% | 103.1% | 103.1% | 101.2% |

As can be seen from the results in Table 7, the appearance of the crystalline form A remained unchanged, and no significant decrease in the content of the crystalline form A and no significant increase in the amount of related substances were observed under severe humidity conditions. These results demonstrate high stability of the crystalline form A under the humidity conditions.

### <6-3> Light stability

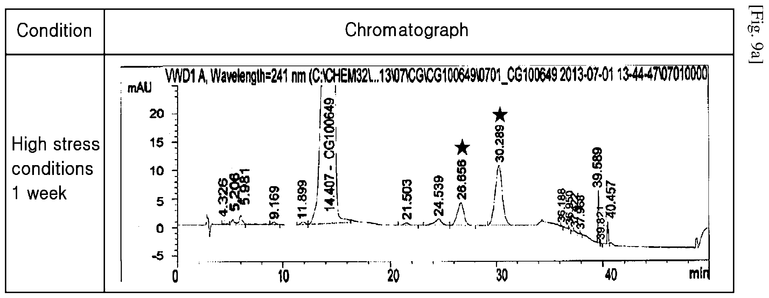

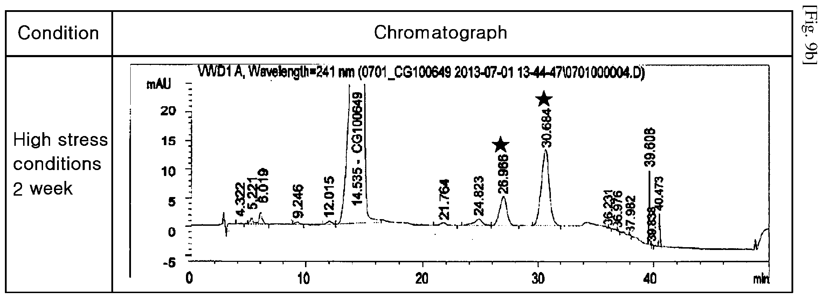

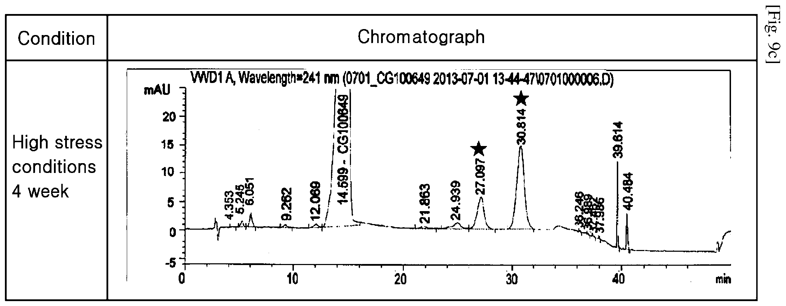

The crystalline form A of Example 1 was evaluated for light stability in the same manner as in Test Example <6-1>. The crystalline form A was filled in a hard capsule (2 mg per capsule) and stored under the light stress conditions shown in Table 8. Thereafter, the appearance of the crystalline form A, the retention time of the major peak, the amounts (%) of related substances, and the compound content were analyzed. The results are shown in Table 8 and Figs. 9a to 9c.

### Table 8

**[Table 8]**

| Packaging material | | PTP | | |
|---|---|---|---|---|
| Stability test conditions(light stress conditions) | | Light*1 week | Light*2 weeks | Light*4 weeks |

| Test item | Criteria | Results | | |
|---|---|---|---|---|
| Appearance | White hard capsule containing white-pale yellow powder and marked with upper green CG649 | Unchanged | Unchanged | Unchanged |
| Peak retention time confirmation | Retention time (RT) of major peak (HPLC) | The same RT | The same RT | The same RT |
| Amounts of related substances | Each < 0.3%, Total < 1.0% | 1.7%, 3.0% | 2.1%, 3.7% | 2.3%, 4.1% |
| Content | 95-105% | 103.4% | 103.1% | 99.1% |

| | | | | |
|---|---|---|---|---|
| * indicates the time when irradiated with light from a white florescent lamp and a UV florescent lamp up to a total illumination of 1.2×10⁶ lux·hr, 200 W·hr/m² | | | | |

As can be seen from Table 8 and Figs. 9a to 9c, the crystalline form A produced related substances exceeding the criteria within 7 days under light stress storage conditions. These results lead to the conclusion that the raw materials should be stored and the formulations should be stored and produced in the dark or in environments protected from exposure to strong light. Light shielding conditions are required in actual processes.

### Examples 2-11: Productions of capsule formulations including the crystalline form A

In order to find optimum pharmaceutical additives suitable for the crystalline form A, the diluents and lubricants shown in Tables 9 and 10 were used to produce capsule formulations.

The Carr's index of each capsule formulation was measured by the Carr's method using a tapped density tester (Erweka, SVM 101) and the angle of repose of each capsule formulation was determined by the fixed funnel method such as the dropping method.

### Table 9

**[Table 9]**

| | | Example 2 | Example 3 | Example 4 | Example 5 | Example 6 |
|---|---|---|---|---|---|---|
| Active ingredient | Crystalline form A of the compound of Formula 1 | 1 | 1 | 1 | 1 | 1 |
| Diluents | Silicified microcrystalline cellulose 50 (Prosolv SMCC 50) | 98 | - | - | - | - |
| | Silicified microcrystalline cellulose 90 (Prosolv SMCC 90) | - | 98 | - | - | - |
| | Microcrystalline cellulose (MCC) | - | - | 98 | - | - |
| | Lactose (Flow lac 100) | - | - | - | 98 | - |
| | Cellactose 80 | - | - | - | - | 98 |
| Lubrica nts | Talc | 1 | 1 | 1 | 1 | 1 |
| | Stearic acid | - | - | - | - | - |
| Total | | 100 | 100 | 100 | 100 | 100 |
| Carr's index | | 29.8 | 22.1 | 33.3 | 14.6 | 24.2 |
| Angle of Repose | | 34.6 | 30.2 | 40.3 | 31.5 | 33.3 |

### Table 10

**[Table 10]**

| | | Example 7 | Example 8 | Example 9 | Example 10 | Example 11 |
|---|---|---|---|---|---|---|
| Active ingredient | Crystalline form A of the compound of Formula 1 | 1 | 1 | 1 | 1 | 1 |
| Diluents | Silicified microcrystalline cellulose 50 (Prosolv SMCC 50) | 98 | - | - | - | - |
| | Silicified microcrystalline cellulose 90 (Prosolv SMCC 90) | - | 98 | - | - | - |
| | Microcrystalli ne cellulose (MCC) | - | - | 98 | - | - |
| | Lactose (Flow lac 100) | - | - | - | 98 | - |
| | Cellactose 80 | - | - | - | - | 98 |
| Lubricants | Talc | - | - | - | - | - |
| | Stearic acid | 1 | 1 | 1 | 1 | 1 |
| Total | | 100 | 100 | 100 | 100 | 100 |
| Carr's index | | 29.9 | 21.9 | 32.8 | 14.6 | 24.1 |
| Angle of Repose | | 34.5 | 32.3 | 37.2 | 31.5 | 33.6 |

As can be seen from the results in Tables 9 and 10, the capsule formulations containing silicified microcrystalline cellulose 50, silicified microcrystalline cellulose 90, microcrystalline cellulose, lactose or Cellactose 80 as a diluent and talc or stearic acid as a lubricant (Examples 2 to 11) had angles of repose in the range of 30 to 40°C and a Carr's index in the range of 21 to 30%. Within these ranges, good flowability of the powders is ensured, thus being suitable for capsule filling. However, the capsule formulations of Examples 7 to 11 using stearic acid as a lubricant had considerably high water contents despite the same experimental conditions as in Examples 2-6. Therefore, it can be concluded that the capsule formulations of Examples 7 to 11 are difficult to produce in a highly humid environment or season, and therefore, the use of talc as a lubricant would be more desirable.

### Test Example 7: Measurement of particle size distributions

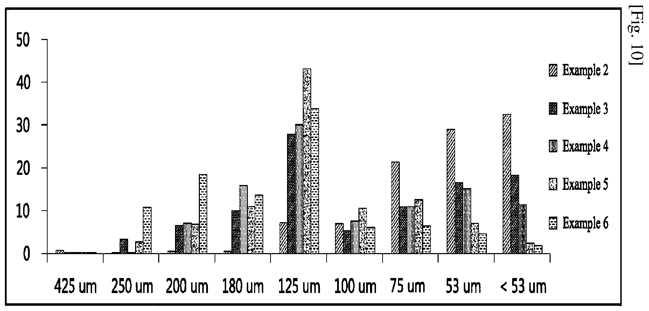

The particle size distributions of the formulations produced in Examples 2-6 were measured using 40-, 60-, 70-, 80-, 120-, 140-, 200-, and 270-mesh standard sieves in accordance with the sieve classification method (method II) described in the standard test methods for particle size of the Korean Pharmacopoeia. The results are shown in Fig. 10.

As shown in Fig. 10, the particle size distributions varied greatly depending on the kind of the diluent and the lubricant used.

Particularly, the formulation of Example 2 produced using silicified microcrystalline cellulose 50 as a diluent showed a uniform particle size distribution in the particle diameter range of less than 125 µm, indicating high mixing uniformity. Silicified microcrystalline cellulose 50 would be more suitable for use in the composition of the present invention due to its high flowability, improved lubricating effects, and ease of mixing compared to other diluents.

### Test Example 8: Analysis of formulation uniformity

The capsule formulations produced in Examples 2-6 were tested for uniformity in accordance with the test method for content uniformity described in the standard test methods for formulation uniformity of the Korean Pharmacopoeia. Six samples were taken from each capsule formulation. The contents of the major ingredient in the samples were measured to determine the average content, standard deviation, and assessed value (AV). The experimental results are shown in Table 11.

### Table 11

**[Table 11]**

| | Average content (%) | Standard deviation | Assessed value (AV) |
|---|---|---|---|
| Example 2 | 99.2 | 1.2 | 2.8 |
| Example 3 | 92.9 | 4.9 | 9.2 |
| Example 4 | 99.1 | 2.2 | 5.3 |
| Example 5 | 90.9 | 5.2 | 12.1 |
| Example 6 | 101.1 | 2.0 | 4.7 |

As can be seen from the results in Table 11, the formulations were found to have good uniformity. Particularly, the formulation of Example 2 had the lowest assessed value (AV), indicating the best uniformity.

## Claims

1. An anti-inflammatory pharmaceutical composition comprising (i) the compound of Formula 1: 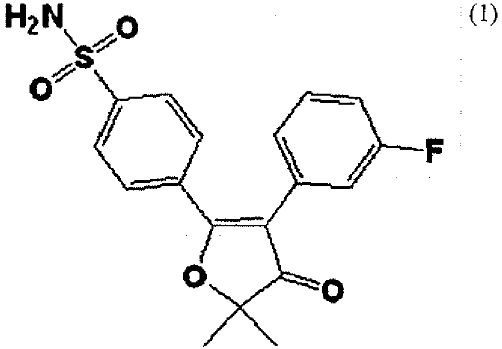 or a pharmaceutically acceptable salt thereof having a 50% volume particle diameter (d_{(0.5)}) of 3 µm to 9 µm measured by laser diffraction, (ii) a pharmaceutically acceptable diluent, and (iii) a pharmaceutically acceptable lubricant,
wherein the compound of Formula 1 exists in crystalline form A that has the results of X-ray diffraction analysis with Cu radiation shown in Table 1 and a differential scanning calorimetry (DSC) profile obtainable by heating at a rate of 10°C/min from 25°C to 220°C, said DSC profile having a peak within a range of from 175.62°C to 178.35°C:
**TABLE 1**
| **2θ** | **Intensity (cps)** |
|---|---|
| **8.40** | **7125** |
| **13.26** | **10050** |
| **14.02** | **2612** |
| **17.70** | **12200** |
| **18.48** | **10388** |
| **19.14** | **7400** |
| **19.84** | **5150** |
| **20.54** | **11750** |
| **22.72** | **2788** |
| **23.56** | **3100** |
| **27.62** | **3088** |
; or
wherein the compound of Formula 1 exists in crystalline form G that has the results of X-ray diffraction analysis with Cu radiation shown in Table 2 and a differential scanning calorimetry (DSC) profile obtainable by heating at a rate of 10°C/min from 25°C to 220°C, said DSC profile having a peak within a range of from 181.06°C to 186.16°C:
**TABLE 2**
| **2θ** | **Intensity (cps)** |
|---|---|
| **11.10** | **3112** |
| **12.66** | **8762** |
| **16.92** | **7812** |
| **18.26** | **18038** |
| **19.48** | **8288** |
| **20.80** | **9775** |
| **22.46** | **4775** |
| **24.02** | **5350** |
| **25.42** | **17138** |
| **27.76** | **4700** |
; or
wherein the compound of Formula 1 exists in a mixture of the crystalline form A and the crystalline form G.

2. The anti-inflammatory pharmaceutical composition according to claim 1, wherein the compound of Formula 1 has a 90% volume particle diameter (d_{(0.9)}) of 10 µm to 100 µm measured by laser diffraction.

3. The anti-inflammatory pharmaceutical composition according to claim 1 or 2, wherein the compound of Formula 1 comprises at least 50% by weight of the crystalline form A.

4. The anti-inflammatory pharmaceutical composition according to any one of claims 1 to 3, wherein the diluent is selected from the group consisting of silicified microcrystalline cellulose, microcrystalline cellulose, cellulose, lactose, and combinations thereof.

5. The anti-inflammatory pharmaceutical composition according to any one of claims 1 to 4, wherein the lubricant is talc or stearic acid.

6. The anti-inflammatory pharmaceutical composition according to any one of claims 1 to 5, wherein the pharmaceutical composition comprises 0.5 to 20% by weight of the compound of Formula 1, 75 to 99% by weight of the diluent, and 0.1 to 5% by weight of the lubricant.

7. The anti-inflammatory pharmaceutical composition according to any one of claims 1 to 6, wherein the pharmaceutical composition comprises 1% by weight of the compound of Formula 1,98% by weight of the diluent, and 1% by weight of the lubricant.

8. A capsule formulation comprising the pharmaceutical composition according to any one of claims 1 to 7.

## Patentansprüche

1. Entzündungshemmende pharmazeutische Zusammensetzung, umfassend (i) die Verbindung der Formel 1: 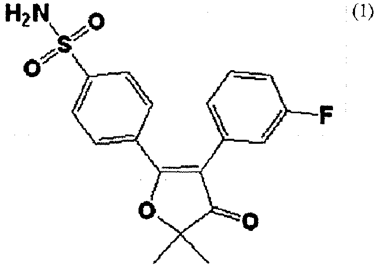 oder ein pharmazeutisch annehmbares Salz davon mit einem 50% Volumenteilchendurchmesser (d_{(0,5)}) von 3 µm bis 9 µm, gemessen durch Laserbeugung, (ii) ein pharmazeutisch annehmbares Streckmittel und (iii) ein pharmazeutisch annehmbares Gleitmittel,
wobei die Verbindung der Formel 1 in kristalliner Form A vorliegt, welche die in Tabelle 1 gezeigten Ergebnisse einer Röntgenbeugungsanalyse mit Cu-Strahlung und ein dynamisches Differenzkalorimetrie(DSC)-Profil, das durch Erwärmen mit einer Rate von 10°C/min von 25°C bis 220°C erhältlich ist, aufweist, wobei das DSC-Profil einen Peak im Bereich von 175,62°C bis 178,35°C hat:
**Tabelle 1**
| **2θ** | **Intensität (cps)** |
|---|---|
| **8,40** | **7125** |
| **13,26** | **10050** |
| **14,02** | **2612** |
| **17,70** | **12200** |
| **18,48** | **10388** |
| **19,14** | **7400** |
| **19,84** | **5150** |
| **20,54** | **11750** |
| **22,72** | **2788** |
| **23,56** | **3100** |
| **27,62** | **3088** |
; oder wobei die Verbindung der Formel 1 in kristalliner Form G vorliegt, welche die in Tabelle 2 gezeigten Ergebnisse einer Röntgenbeugungsanalyse mit Cu-Strahlung und ein dynamisches Differenzkalorimetrie(DSC)-Profil, das durch Erwärmen mit einer Rate von 10°C/min von 25°C bis 220°C erhältlich ist, aufweist, wobei das DSC-Profil einen Peak im Bereich von 181,06°C bis 186,16°C hat:
**Tabelle 2**
| **2θ** | **Intensität (cps)** |
|---|---|
| **11,10** | **3112** |
| **12,66** | **8762** |
| **16,92** | **7812** |
| **18,26** | **18038** |
| **19,48** | **8288** |
| **20,80** | **9775** |
| **22,46** | **4775** |
| **24,02** | **5350** |
| **25,42** | **17138** |
| **27,76** | **4700** |
; oder wobei die Verbindung der Formel 1 in einer Mischung der kristallinen Form A und der kristallinen Form G vorliegt.

2. Entzündungshemmende pharmazeutische Zusammensetzung gemäß Anspruch 1, wobei die Verbindung der Formel 1 einen 90% Volumenteilchendurchmesser (d_{(0,9)}) von 10 µm bis 100 µm, gemessen durch Laserbeugung, aufweist.

3. Entzündungshemmende pharmazeutische Zusammensetzung gemäß Anspruch 1 oder 2, wobei die Verbindung der Formel 1 mindestens 50 Gew.-% der kristallinen Form A umfasst.

4. Entzündungshemmende pharmazeutische Zusammensetzung gemäß einem der Ansprüche 1 bis 3, wobei das Streckmittel ausgewählt ist aus der Gruppe bestehend aus verkieselter mikrokristalliner Cellulose, mikrokristalliner Cellulose, Cellulose, Lactose und Kombinationen davon.

5. Entzündungshemmende pharmazeutische Zusammensetzung gemäß einem der Ansprüche 1 bis 4, wobei das Gleitmittel Talk oder Stearinsäure ist.

6. Entzündungshemmende pharmazeutische Zusammensetzung gemäß einem der Ansprüche 1 bis 5, wobei die pharmazeutische Zusammensetzung 0,5 bis 20 Gew.-% der Verbindung der Formel 1, 75 bis 99 Gew.-% des Streckmittels und 0,1 bis 5 Gew.-% des Gleitmittels umfasst.

7. Entzündungshemmende pharmazeutische Zusammensetzung gemäß einem der Ansprüche 1 bis 6, wobei die pharmazeutische Zusammensetzung 1 Gew.-% der Verbindung der Formel 1, 98 Gew.-% des Streckmittels und 1 Gew.-% des Gleitmittels umfasst.

8. Kapselformulierung, umfassend die pharmazeutische Zusammensetzung gemäß einem der Ansprüche 1 bis 7.

## Revendications

1. Composition pharmaceutique anti-inflammatoire comprenant (i) le composé de la formule 1: 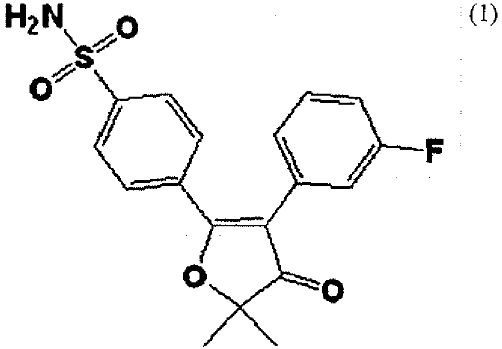 ou un sel pharmaceutiquement acceptable de celui-ci ayant un diamètre des particules volumique de 50% (d_{(0,5)}) de 3 µm à 9 µm mesuré par diffraction laser, (ii) un diluant pharmaceutiquement acceptable, et (iii) un lubrifiant pharmaceutiquement acceptable,
le composé de la formule 1 existant sous forme cristalline A dont les résultats de l'analyse par diffraction des rayons X avec le rayonnement CU sont montrés dans le tableau 1 et un profil de la calorimétrie par balayage différentiel (DSC) pouvant être obtenu par une chauffage à une vitesse de 10° C /min de 25° C à 220° C, ledit profil DSC ayant un pic dans l'intervalle de 175,62° C à 178,35° C:
**TABLEAU 1**
| **2θ** | **Intensité (cps)** |
|---|---|
| **8.40** | **7125** |
| **13.26** | **10050** |
| **14.02** | **2612** |
| **17.70** | **12200** |
| **18.48** | **10388** |
| **19.14** | **7400** |
| **19.84** | **5150** |
| **20.54** | **11750** |
| **22.72** | **2788** |
| **23.56** | **3100** |
| **27.62** | **3088** |
; ou
le composé de la formule 1 existant sous forme cristalline G dont les résultats de l'analyse par diffraction des rayons X avec le rayonnement CU sont montrés dans le tableau 2 et un profil de la calorimétrie par balayage différentiel (DSC) pouvant être obtenu par une chauffage à une vitesse de 10° C /min de 25° C à 220° C, ledit profil DSC ayant un pic dans l'intervalle de 181,06° C à 186,16° C:
**TABLEAU 2**
| **2θ** | **Intensité (cps)** |
|---|---|
| **11.10** | **3112** |
| **12.66** | **8762** |
| **16.92** | **7812** |
| **18.26** | **18038** |
| **19.48** | **8288** |
| **20.80** | **9775** |
| **22.46** | **4775** |
| **24.02** | **5350** |
| **25.42** | **17138** |
| **27.76** | **4700** |
; ou
le composé de la formule 1 existant dans un mélange de la forme cristalline A et la forme cristalline G.

2. Composition pharmaceutique anti-inflammatoire selon la revendication 1, le composé de la formule 1 ayant un diamètre des particules volumique de 90% (d_{(0,9)}) de 10 µm à 100 µm mesuré par diffraction laser.

3. Composition pharmaceutique anti-inflammatoire selon la revendication 1 ou 2, le composé de la formule 1 comprenant au moins 50% en poids de la forme cristalline A.

4. Composition pharmaceutique anti-inflammatoire selon l'une quelconque des revendications 1 à 3, le diluant étant choisi parmi le groupe constitué par une cellulose microcristalline silicifiée, une cellulose microcristalline, une cellulose, un lactose et leurs combinaisons.

5. Composition pharmaceutique anti-inflammatoire selon l'une quelconque des revendications 1 à 4, le lubrifiant étant du talc ou de l'acide stéarique.

6. Composition pharmaceutique anti-inflammatoire selon l'une quelconque des revendications 1 à 5, la composition pharmaceutique comprenant 0,5 à 20 % en poids de la formule 1, 75 à 99% en poids du diluant, et 0,1 à 5% en poids du lubrifiant.

7. Composition pharmaceutique anti-inflammatoire selon l'une quelconque des revendications 1 à 6, la composition pharmaceutique comprenant 1% en poids du composé de la formule 1, 98% en poids du diluant, et 1% en poids du lubrifiant.

8. Formulation de capsule comprenant la composition pharmaceutique selon l'une quelconque des revendications 1 à 7.
